# EUROPEAN PATENT SPECIFICATION

(11) **EP 4 375 507 B1**
(45) Date of publication and mention of the grant of the patent: **11.03.2026**
(21) Application number: 22209732.1
(22) Date of filing: 25.11.2022
(51) Int. Cl.: F04B 7/06, F04B 9/04, F04B 9/105, F04B 13/00, F04B 19/00

(54) **MICROPUMP**
MIKROPUMPE
MICROPOMPE

(43) Date of publication of application: 29.05.2024
(73) Proprietor: Sensile Medical AG, 4600 Olten (CH)
(72) Inventor: MUELLER, Matthias, 4614 Hägendorf (CH)
(74) Representative: reuteler & cie SA

(56) References cited:
- EP-B1- 3 732 375
- WO-A1-2009/069063
- WO-A1-2021/156574
- DE-A1- 3 630 528
- US-A- 5 102 310
- US-A1- 2008 187 449
- US-A1- 2020 309 104
- US-B2- 11 105 321

## Description

### TECHNICAL FIELD

The present invention relates to a micropump. The micropump may be used for dispensing small quantities of liquid, in particular for use in medical applications, for instance in a drug delivery device. A micropump related to the invention may also be used in non-medical applications that require high precision delivery of small quantities of liquid.

### DESCRIPTION OF RELATED ART

A micropump for delivering small quantities of liquid that may in particular be used in medical and non-medical applications is described in EP1803934 and in EP1677859. The micropump described in the aforementioned documents includes a rotor with first and second axial extensions of different diameters that engage with first and second seals of the stator to create first and second valves that open and close liquid communication across the respective seal as a function of the angular and axial displacement of the rotor. A pump chamber is formed between the first and second seals of the stator whereby the pumped volume of liquid per rotation cycle of the rotor is a function of both the difference in diameters between the first and second rotor axial extensions and the axial displacement of the rotor that is effected by a cam system as a function of the angular position of the rotor with respect to the stator.

The ability to pump small quantities of liquids by continuous rotation of a rotor is advantageous in many situations. In view of the small volume of liquid pumped per rotation cycle, the rotary drive output may rotate at speeds that are generally greater than the speed of a screw mechanism for advancing a piston of a piston pump. The rotary drive is simple to control and avoiding a piston mechanism allows the pump to be very compact. Also, the pump module may be made of low cost disposable parts, for instance of injected polymers.

In certain applications, in particular for pumping liquids containing molecules that are sensitive to friction, the friction between the rotor shaft and the valve seals of a pump as described in EP1803934 may however be undesirable. This could for instance be a problem with large molecules such as certain proteins that are sensitive to shear stress.

The aforementioned problem could be overcome by provision of other pump systems, in particular piston pumps or pumps comprising cartridges with a plunger that is advanced by a piston rod. Such pump systems are however not very economical and not very compact in view of the length of the piston mechanism. Reliability and safety of piston pump systems may also be an issue because they do not inherently block direct fluid communication between the liquid container and the outlet of the pump system.

Another micropump for delivering small quantities of liquid that may in particular be used in medical and non-medical applications is described in EP3732375. The micropump described in this document includes rotor axially and rotatably movable relative to a stator. The rotor comprises a port arranged on the outer surface of the rotor shaft. The port is arranged to overlap at least partially the inlet over a rotational angle of the rotor corresponding to a pump intake phase, and arranged to overlap at least partially the outlet over a rotational angle of the rotor corresponding to a pump expel phase. The stator comprises a rotor shaft receiving cavity, the inlet and outlet fluidly connected to the rotor shaft receiving cavity, the rotor comprising a shaft received in the rotor shaft receiving cavity. The rotor shaft comprises a cavity receiving a piston portion of the stator therein to form a piston chamber, a seal mounted between the piston portion and inner sidewall of the cavity to sealingly close an end of the piston chamber. The rotor further comprises a port fluidly connecting the piston chamber to an outer surface of the rotor shaft. However, the pump of EP3732375 does not disclose a non-axisymmetric indent formed between the radial surface and the end face of the piston portion; moreover, this document does not disclose an arrangement in which the piston portion is shaped, and the membrane is configured, to elastically conform the membrane in a form fitting elastically tensioned manner over the indent, the end face and the radial surface of the piston portion during an entire pump cycle.

In the pump of EP3732375, the liquid being pumped is still between portions of the rotor and stator that move relative to each other during a pump cycle, which is potentially harmful for delicate substances being pump.

### SUMMARY OF THE INVENTION

In view of the foregoing, an object of the invention is to provide a micropump that is capable of pumping small quantities of liquid in a reliable and safe manner.

It is advantageous in certain applications to provide a micropump that does not apply shear stress on the liquid being pumped.

It is advantageous to provide a micropump that does not contaminate the liquid being pumped.

It is advantageous to provide a micropump that is very compact.

It is advantageous to provide a micropump that has a small dead volume.

It is advantageous to provide a micropump that is economical to manufacture and that may be incorporated in a disposable non-reusable component, such as in a disposable part of a drug delivery device.

Objects of the invention are achieved by a micropump according to claim 1.

Disclosed herein is a pump comprising a stator and a rotor axially and rotatably movable relative to the stator, the stator comprising a piston chamber comprising a radial surface, an inlet comprising an inlet mouth formed in the radial surface and an outlet comprising an outlet mouth formed in the radial surface, the rotor comprising a piston portion comprising a radial surface, an end face and a non-axisymmetric indent formed between the radial surface and the end face of the piston portion, wherein the indent is arranged to overlap at least partially the inlet mouth over a range of angular positions of the rotor relative to the stator corresponding to a pump intake phase, and arranged to overlap at least partially the outlet mouth over a range of angular positions of the rotor relative to the stator corresponding to a pump expel phase, and wherein the radial surface of the piston portion is configured to close the outlet mouth during the pump intake phase, the inlet mouth during the pump expel phase and both the inlet and outlet mouths between the pump intake and expel phases. The stator comprises a flexible elastic membrane sealingly separating the piston portion from the piston chamber.

In an advantageous embodiment, the flexible elastic membrane comprises a single layer or a plurality of layers of a flexible material.

In an advantageous embodiment, the flexible material is silicone rubber or thermoplastic elastomer.

In an advantageous embodiment, the thickness of the single layer or each of the plurality of layers is between 0.1 mm and 1 mm.

In an advantageous embodiment, the single layer or each of the plurality of layers is made by injection molding or by compression molding.

In an advantageous embodiment, the pump comprises a lubricant between at least two of the plurality of layers.

In an advantageous embodiment, the pump comprises a lubricant between the piston portion and the flexible elastic membrane.

In an advantageous embodiment, the piston portion is shaped, and the membrane is configured, to elastically conform the membrane in a form fitting elastically tensioned manner over the indent, the end face and the radial surface of the piston portion during an entire pump cycle.

In an advantageous embodiment, the ratio of the piston stroke to the diameter of the piston portion is smaller than 0.5, preferably, smaller than 0.2.

In an advantageous embodiment, the stator comprises a cap portion and a base portion and wherein a rim portion of the membrane is fastened between the cap portion and the base portion.

In an advantageous embodiment, the cap portion is fastened to the base portion by a snap lock arrangement.

In an advantageous embodiment, the pump further comprises sealing ribs protruding from the rotor or the surface of the piston chamber configured to sealingly pinch the flexible elastic membrane and seal the piston chamber at least above the inlet and outlet mouths.

In an advantageous embodiment, the sealing ribs include at least one lower sealing rib protruding from the rotor configured to sealingly pinch the elastic membrane below the outlet mouth and above the inlet mouth during an open phase of the inlet, respectively below the inlet mouth and above the outlet mouth during an open phase of the outlet.

In an advantageous embodiment, the sealing ribs include sealing ribs protruding from the radial surface of the piston chamber surrounding the inlet mouth and the outlet mouth.

In an advantageous embodiment, the pump comprises a camming system that defines an axial position of the rotor with respect to the stator as a function of angular position of the rotor with respect to the stator.

Further objects and advantageous features of the invention will be apparent from the claims, from the detailed description, and annexed drawings, in which:

### BRIEF DESCRIPTION OF THE DRAWINGS

Figure 1 is a schematic perspective view of a micropump according to an embodiment of the invention;
Figure 2 is a schematic perspective view of a rotor of a micropump according to an embodiment of the invention;
Figure 3 is a schematic cross-sectional view of a micropump according to an embodiment of the invention;
Figure 4 is a detail portion illustrating an inlet or outlet of the micropump in a closed phase of the pump cycle;
Figure 5 is a view similar to figure 4 of a variant;
Figures 6a-6h are schematic cross-sectional views (upper panels) and schematic top views (lower panels) illustrating eight different rotor positions in a pump cycle of the micropump according to an embodiment of the invention, from intake to expulsion of liquid;
Figure 7 is a graph illustrating the sealing profile of the indent at axial positions z and angular positions *φ* of the inner surface of the piston chamber for rotor positions at the opening and closing of the inlet and outlet valves.

### DETAILED DESCRIPTION OF EXEMPLARY EMBODIMENTS

Referring to the figures, a micropump 1 according to embodiments of the invention comprises a stator 4 and a rotor 6 coupled to a rotary drive 2 that rotates the rotor 6 about an axis A relative to the stator 4. The rotor 6 is also axially movable relative to the stator 4, the axial direction z being aligned with the axis of rotation A.

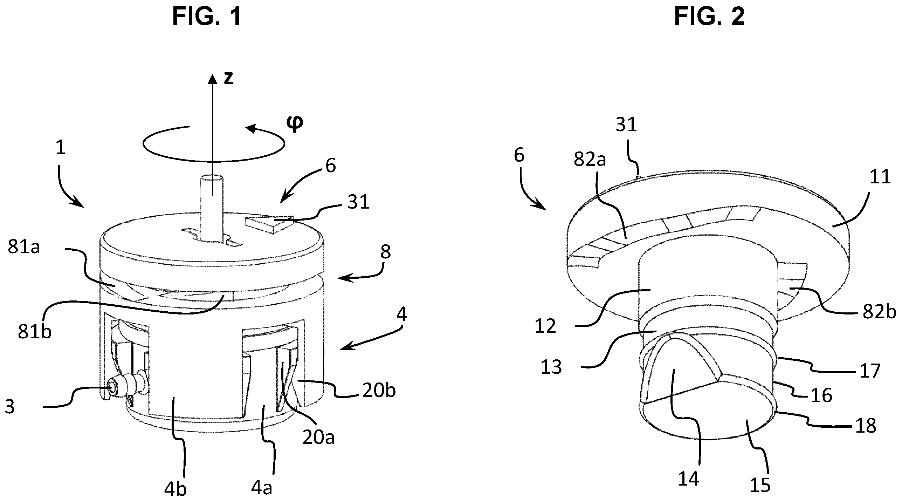

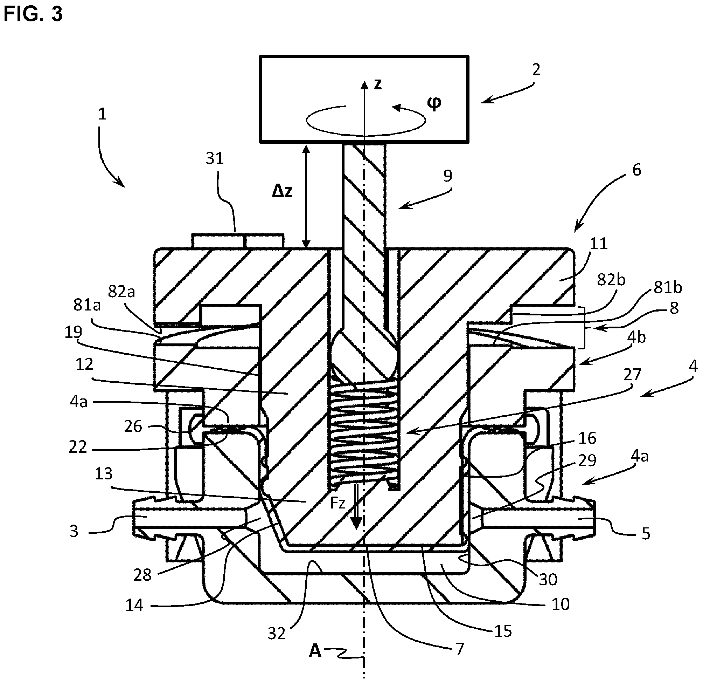

The rotary drive 2 is coupled to the rotor 6 via a coupling 9 that allows axial displacement *Δz* of the rotor relative to the motor yet couples the output of the rotary drive in rotation to the rotor. The coupling 9 comprises a biasing mechanism 27, for instance in the form of a spring, such as a coil spring that applies an axial force Fz towards the stator 4. The coupling 9 may be integrated in a cavity of the rotor 6 as illustrated in Figs. 1, 3, and 6.

The rotor and stator comprise a camming system 8 that defines an axial position z of the rotor with respect to the stator as a function of angular position *φ* of the rotor with respect to the stator. The camming system 8 may comprise a cam track 81a biased against a complementary cam follower 82a, the biasing mechanism 27 ensuring that the cam follower presses against the cam track 81a. The cam track 81a has a profile P that defines the axial position of the rotor relative to the stator as a function of the angular position of the rotor relative to the stator. The camming system 8 may comprise one or more pairs of cam tracks 81a, 81b and complementary cam followers 82a, 82b.

In the embodiments illustrated, the camming system 8 comprises two pairs of cam tracks 81a, 81b and complementary cam followers 82a, 82b. The cam tracks 81a, 81b are formed on a rim of the stator 4 whereas the complementary cam followers 82a, 82b are provided on a head 11 of the rotor 6. The skilled person will however appreciate that one or more cam tracks may be provided on the rotor and the corresponding cam followers on the stator.

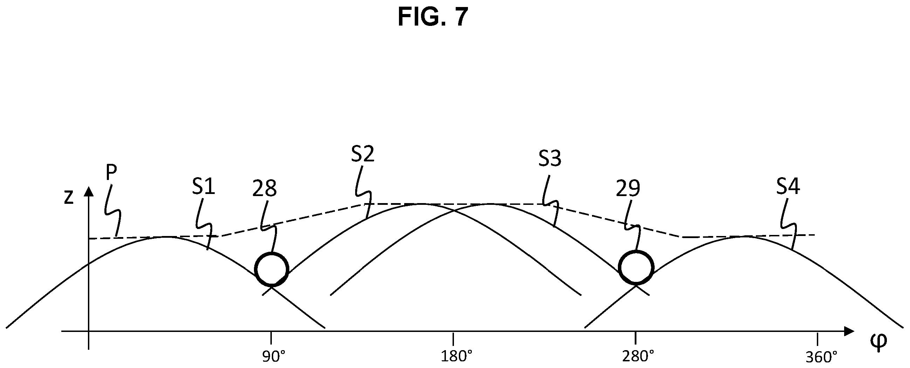

An example of a cam track profile P developed over a 360° rotation cycle is illustrated in figure 7.

In the illustrated embodiments, the biasing mechanism 27 and camming system 8 form together an axial displacement system defining the axial displacement of the rotor relative to the stator as a function of the rotor's angular position, however other axial displacement systems may be implemented.

For instance, axial displacement may be effected by an electromagnetic actuator coupled to the rotary drive, or may be provided by means of a drive that outputs both a rotational and an axial movement. The rotor 6 comprises a shaft 12 comprising a piston portion 13 rotatably and slidably inserted in a piston chamber 10 of the stator 4. The piston chamber 10 has an inner surface comprising a radial surface 30 and an end face 32. The piston portion 13 has an outer surface comprising a radial surface 16 and an end face 15. The inner surface of the piston chamber 10 is in close proximity to the outer surface of the piston portion 13 when the piston portion 13 is fully inserted in the piston chamber 10 at an angular position of the rotor at which the end face 15 of the piston portion 13 abuts the end face 32 of the piston chamber 10. The radial surfaces 30, 16 of the piston chamber 10 and the piston portion 13 are radial surfaces of right circular cylinders. In the illustrated embodiments, the end faces 32, 15 of the piston chamber 10 and the piston portion 13 are planar. However, the end faces 32, 15 of the piston chamber 10 and the piston portion 13 may have a different shap, e.g., they may have the shape of a different surface of revolution generated by the revolution of a curve about the axis A of shaft of the rotor, in particular, a spherical shape.

The stator 4 comprises an inlet 3 and an outlet 5 in fluid communication with the piston chamber 10. It will be appreciated that inlet may become an outlet and the outlet an inlet respectively depending on the direction of rotation of the rotor. In a variant, the pump may be reversible for bidirectional pumping of liquid through the pump, the direction depending on the direction of rotation of the rotor. Alternatively, the pump may be configured to be unidirectional, allowing rotation of the rotor only in one direction for pumping of fluid only in one direction through the pump.

In the illustrated embodiments, both the inlet and the outlet extend through a sidewall of the stator, however it may be appreciated that the inlet and/or outlet could be formed as a channel of various shapes extending within a body of the stator for coupling at various positions of the stator to a liquid source or a liquid output device, as a function of the application and desired configuration.

A micropump according to embodiments of the invention may advantageously be used in a drug delivery device for administering a liquid drug to a patient. The outlet may therefore be connected to a needle, for transcutaneous administration of a drug, or to a catheter or other liquid conduit connected to the patient. The inlet may be connected to a drug vial, cartridge or other liquid drug source.

The stator 4 further comprises a membrane 7 fluidly separating the piston portion 13 of the rotor 6 from the piston chamber 10. The membrane 7 is made of a flexible material, which is impermeable to the liquid intended to be pumped with the micropump, e.g., impermeable to water. For example, the membrane may be made of silicone rubber or thermoplastic elastomer. The membrane 7 may comprise a single layer or a plurality of layers of the flexible material. The thickness of the single layer or each of the plurality of layers of the membrane may be less than 1 mm, but more than 0.1 mm.

The micropump may further comprise a lubricant such as silicone- mineral- or synthetic oil or grease between the piston portion 13 and the membrane 7 to reduce the friction between the piston portion 13 and the membrane 7. In case the membrane 7 comprises a plurality of layers of the flexible material, a lubricant may be added between the layers. The single layer or each of the plurality of layers of the membrane may be made of a flat flexible elastic foil. In a variant, the single layer or each of the plurality of layers of the membrane may be made by injection molding to obtain a membrane with a shape in unstressed condition similar to the shape of the outer surface 16 of the piston portion 13.

The membrane 7 has a sufficiently high prestress to ensure that the membrane 7 conforms with the outer surface 16 of the piston portion 13 during the entire pump cycle.

The membrane 7 stretches and contracts with the axial displacement of the piston portion 13. It is advantageous to have a small relative expansion of the membrane 7 in order to have a small movement of the portion of the membrane that covers the end face 15 of the piston portion. Therefore, it is advantageous to have a small ratio of the piston stroke to the diameter of the piston portion. For example, the ratio of the piston stroke to the diameter of the piston portion may be smaller than 0.5, in particular, smaller than 0.2.

In the illustrated embodiments, the stator 4 comprises a cap portion 4b and a base portion 4a. The cap portion 4b of the stator 4 may be fixed to the base portion 4a by various fixing means such as welding, adhesive bonding, clamping, screwing and other *per se* known bonding or mechanical fastening means. In the illustrated embodiment, the cap portion 4b is fastened to the base portion 4a of the stator 4 by a snap lock arrangement comprising a fixing element 20b in the form of a locking latch, for instance on the cap portion 4b and a complementary fixing element 20a in the form of a locking shoulder 20a extending radially outward on the base portion. The latches and shoulders may be inverted such that the locking latches are on the base portion and the locking shoulder on the cap portion.

The cap portion 4b has a passage for the rotor shaft 19.

In the illustrated embodiments, a rim portion of the membrane 7 is clamped between a clamping surface 21 of the cap portion 4b and a clamping surface 22 of the base portion 4a. The clamping surface 21 of the cap portion 4b and the clamping surface 22 of the base portion 4a are pressed together by the snap lock arrangement. The clamping surfaces 21, 22 of the upper and base portions have complementary ridges and grooves for squeezing the membrane 7 between the clamping surfaces 21, 22.

Additionally, the membrane 7 might have a collar 26 at its periphery arranged outside the clamping surfaces 21, 22 for additional prevention for the membrane to slip through the clamping surfaces.

In a variant, the cap portion 4b and the base portion 4a of the stator 4 are integrally formed as a single-piece element and the membrane 7 is fastened to the stator 4 using a fastening means such as an adhesive or by welding or by being fixed to a rigid ring support that is assembled to the stator and fixed by mechanical means or bonded or welded to the stator. In a variant the sealing membrane may also be integrally molded with the stator or a portion of the stator in a two component injection molding process.

The piston portion 13 further comprises a non-axisymmetric indent 14 for instance in the form of a taper or chamfer between the end face 15 and the radial surface 16 of the piston portion 13. The taper or chamfer may have a substantially flat (planar) surface, or in variants may have a convex curved or multifaceted surface, provided that the indent removes material from a portion of the corner of the piston. The indent has an axial height that extends at least to an inlet 3 and outlet 4 of the stator so that the indent passes by the inlet mouth 28 respectively by the outlet mouth 29 during a rotation of the piston.

The inlet 3 is in fluid communication with an inlet mouth 28 in the radial surface 30 of the piston chamber 10. The outlet 5 is in fluid communication with an outlet mouth 29 in the radial surface 30 of the piston chamber 10 diametrically opposite the inlet mouth 28.

An edge between the radial surface 16 and the end face 15 of the piston portion 13 and an edge of the indent 14 defines a circumferential sealing profile between the piston portion 13 and the piston chamber 10. The inlet and outlet mouths 28, 29 are axially positioned within the axial positions of the sealing profile defined by the edge of the indent 14. The sealing profile of the indent 14 is displaced in axial direction according to the cam track profile P as a function of the angular position of the rotor relative to the stator. The sealing profile of the indent 14 opens and closes the inlet and outlet mouths 28, 29 and thus enables and disables fluid communication between the piston chamber 10 and the inlet 3 and outlet 5, respectively. Thus, the combination of the inlet and outlet mouths 28, 29 with the sealing profile of the indent 14 act as inlet and outlet valves, which open and close as a function of angular position of the rotor relative to the stator.

The sealing profile of the indent 14 at axial positions z and angular positions *φ* of the inner surface of the piston chamber 10 is illustrated in figure 7 for rotor positions at the opening and closing of the inlet and outlet valves. The origin of the coordinate system is defined by the center of the indent 14 at an angular position in the middle between the angular positions of the inlet and outlet mouths when the piston portion 13 is fully inserted in the piston chamber 10. The angular position of the center of the indent 14 may be marked by an indicator 31 on the head 11 of the rotor 6.

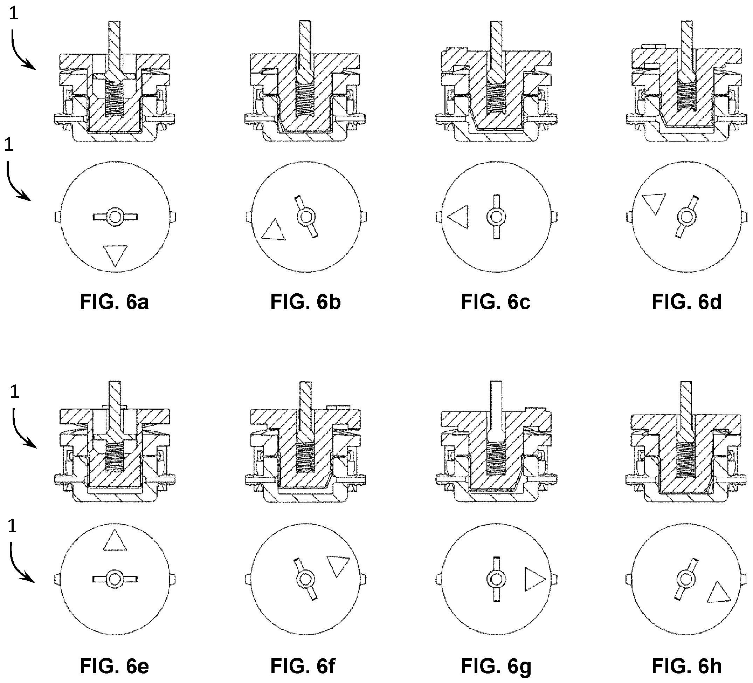

Both the inlet and outlet valves are closed at the angular position of 0° as illustrated in figure 6a. At a first angle *φ1* between 0° and 90°, a first side of the sealing profile of the indent 14 reaches the inlet mouth 28 (curve S1 in figure 7) and the inlet valve starts to open as illustrated in figure 6b. After the inlet valve is at least partially open, the piston portion starts to move in axial direction with increasing angle and liquid is pumped from the inlet 3 into the piston chamber 10. The inlet valve starts to close when a second side of the sealing profile of the indent 14 reaches the inlet mouth 28 as illustrated in figure 6d. The axial displacement of the rotor and thus the uptake of liquid is stopped before the inlet valve is fully closed at a second angle *φ2* between 90° and 180° (curve S2 in figure 7). Both the inlet and outlet valves are closed at the angular position of 180° as illustrated in figure 6e. At a third angle *φ3* between 180° and 270°, the first side of the sealing profile of the indent 14 reaches the outlet mouth 29 and the outlet valve starts to open (curve S3 in figure 7). After the outlet valve is at least partially open, the piston portion starts to move in opposite axial direction with increasing angle and liquid is pumped from the piston chamber 10 to the outlet 5 as illustrated in figure 6f-h. The outlet valve starts to close when a second side of the sealing profile of the indent 14 reaches the outlet mouth 29. The axial displacement of the rotor and thus the expulsion of liquid is stopped before the outlet valve is fully closed at a fourth angle *φ4* between 270° and 360° (curve *S4* in figure 7).

The dead volume inside the piston chamber 13 is the volume of the indent 14 relative to the axisymmetric profile of the piston, provided that the piston portion is fully inserted into the piston chamber at the angular position of 0°.

In an embodiment illustrated in figures 2-4, the piston portion 13 of the rotor 6 comprises a lower circumferential sealing rib 18 radially outwardly extending from the piston portion 13 along the edge of the indent 14 and the edge between the end face 15 and the radial surface 16 of the piston portion 13. The membrane 7 is squeezed (pinched) between the lower circumferential sealing rib 18 and the radial surface 30 of the piston chamber 10. The lower circumferential sealing rib 18 defines a circumferential sealing profile between the piston portion 13 and the piston chamber 10. The piston portion 13 further comprises an upper circumferential sealing rib 17 radially outwardly extending from the piston portion 13. The upper circumferential sealing rib 17 is arranged axially proximally above the inlet and outlet mouths (28, 29), i.e., in axial direction closer to the rotor head than the inlet and outlet mouths throughout the pump cycle. The upper and lower circumferential sealing rib 17, 18 improve the sealing of the inlet and outlet valves and between the piston portion 13 and the piston chamber 10. The piston portion 13 may further comprise one or more vertical sealing ribs radially outwardly extending from the piston portion 13 connecting the upper circumferential sealing rib 17 with the lower circumferential sealing rib 18 in axial direction to avoid leaking between the inlet and outlet when both valves are closed. For example, a vertical sealing rib may be arranged opposite the center of the indent.

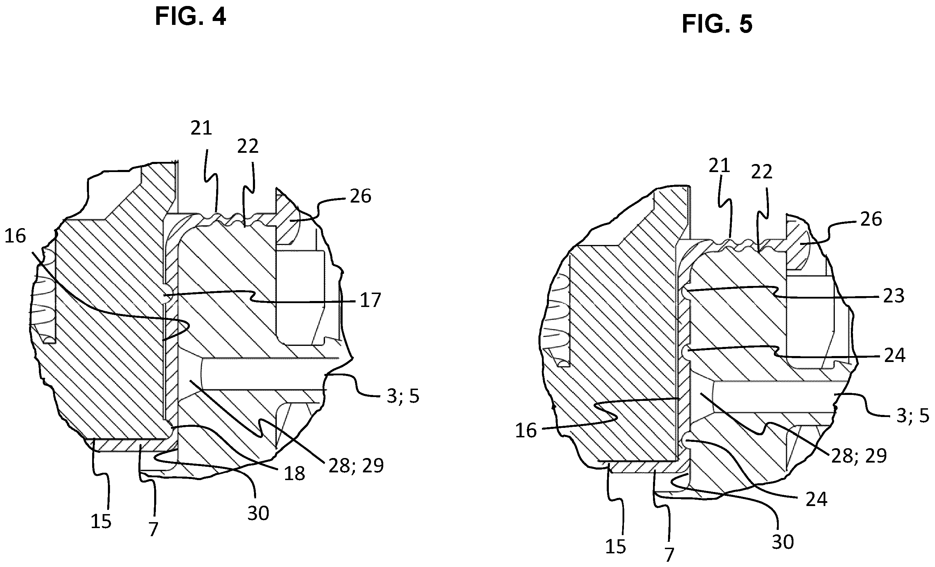

In a variant illustrated in figure 5, the sealing of the inlet and outlet valves and between the piston portion and the piston chamber is improved by sealing ribs added to the piston chamber. In this variant, the piston chamber 10 comprises a first sealing rib radially inwardly extending from the radial surface 30 of the piston chamber 10 encircling the inlet mouth 28 and a second sealing rib 24 radially inwardly extending from the radial surface 30 of the piston chamber 10 encircling the outlet mouth 29. The piston chamber 10 further comprises a circumferential sealing rib 23 radially inwardly extending from the radial surface 30 of the piston chamber 10. The circumferential sealing rib 23 of the piston chamber 10 is arranged axially proximally above the inlet and outlet mouths, i.e., closer to the rotor head than the inlet and outlet mouths throughout the pump cycle.

### List of features

*Micropump 1*
***Stator 4***
   *Cap portion 4b*
      *Passage for rotor shaft 19*
      *Fixing element 20b*
         *e.g. locking latch*
      *Clamping surface 21*
   *Base portion 4a*
      *Inlet 3*
         *Inlet mouth 28*
      *Outlet 5*
         *Outlet mouth 29*
      *Piston chamber 10*
         *Radial surface 30*
         *End face 32*
      *Fixing element 20a*
         *e.g..locking shoulder*
      *Clamping surface 22*
      *Circumferential sealing rib 23*
      *Sealing ribs encircling inlet and outlet mouths 24*
   *Membrane 7*
      *Collar 26*
***Rotor 6***
   *Head 11*
      *Indicator 31*
   *Shaft 12*
      ***Piston portion 13***
      *Indent 14*
      *End face 15*
      *Radial surface 16*
      *Upper circumferential sealing rib 17*
      *Lower circumferential sealing rib 18*
***Inlet valve V1***
***Outlet valve V2***
***Rotor positions***
   *Sealing profile of the indent at opening*/*closing of inlet valve S1, S2*
   *Sealing profile of the indent at opening*/*closing of outlet valve S3, S4*
***Axial displacement system***
   ***Camming system 8***
      *Cam tracks on stator 81a, 81b*
      *Complementary cam followers on rotor 82a, 82b*
   *Coupling 9*
      *Biasing Mechanism 27*
***Rotary drive 2***

## Claims

1. A micropump (1) comprising a stator (4) and a rotor (6) axially and rotatably movable relative to the stator, the stator (4) comprising a piston chamber (10) comprising a radial surface (30), an inlet (3) comprising an inlet mouth (28) formed in the radial surface and an outlet (5) comprising an outlet mouth (29) formed in the radial surface, the rotor (6) comprising a piston portion (13) comprising a radial surface (16), an end face (15) and a non-axisymmetric indent (14) formed between the radial surface (16) and the end face (15) of the piston portion (13), wherein the indent (14) is arranged to overlap at least partially the inlet mouth (28) over a range of angular positions of the rotor relative to the stator corresponding to a pump intake phase, and arranged to overlap at least partially the outlet mouth (29) over a range of angular positions of the rotor relative to the stator corresponding to a pump expel phase, and wherein the radial surface (16) of the piston portion (13) is configured to close the outlet mouth (29) during the pump intake phase, the inlet mouth (28) during the pump expel phase and both the inlet and outlet mouths (28) between the pump intake and expel phases, wherein the stator (4) comprises a flexible elastic membrane (7) sealingly separating the piston portion (13) from the piston chamber (10), wherein the piston portion (13) is shaped, and the membrane is configured, to elastically conform the membrane in a form fitting elastically tensioned manner over the indent (14), the end face (15) and the radial surface (16) of the piston portion (13) during an entire pump cycle.

2. The micropump according to claim 1, wherein the flexible elastic membrane (7) comprises a single layer or a plurality of layers of a flexible material.

3. The micropump according to the directly preceding claim, wherein the flexible material is silicone rubber or thermoplastic elastomer.

4. The micropump according to any of the two directly preceding claims, wherein the thickness of the single layer or each of the plurality of layers is between 0.1 mm and 1 mm.

5. The micropump according to any of the three directly preceding claims wherein the flexible elastic membrane (7) comprises said plurality of layers, and the pump comprises a lubricant between at least two of the plurality of layers.

6. The micropump according to any preceding claim, comprising a lubricant between the piston portion (13) and the flexible elastic membrane (7).

7. The micropump according to any preceding claim, wherein the ratio of the piston stroke to the diameter of the piston portion (13) is smaller than 0.5, preferably, smaller than 0.2.

8. The micropump according to any preceding claim, wherein the stator (4) comprises a cap portion (4b) and a base portion (4a) and wherein a rim portion of the membrane (7) is fastened between the cap portion (4b) and the base portion (4a).

9. The micropump according to the directly preceding claim, wherein the cap portion (4b) is fastened to the base portion (4a) by a snap lock arrangement.

10. The micropump according to any preceding claim, further comprising sealing ribs (17, 18, 23, 24) protruding from the rotor (6) or the surface of the piston chamber (10) configured to sealingly pinch the flexible elastic membrane (7) and seal the piston chamber at least above the inlet and outlet mouths.

11. The micropump according to the directly preceding claim, wherein the sealing ribs include at least one lower sealing rib (18) protruding from the rotor configured to sealingly pinch the elastic membrane (7) below the outlet mouth (29) and above the inlet mouth (28) during an open phase of the inlet (3), respectively below the inlet mouth (28) and above the outlet mouth (29) during an open phase of the outlet (5).

12. The micropump according to claim 10 wherein the sealing ribs include sealing ribs (24) protruding from the radial surface (30) of the piston chamber (10) surrounding the inlet mouth (28) and the outlet mouth (29).

13. The micropump according to any preceding claim, comprising a camming system (8) that defines an axial position (z) of the rotor (6) with respect to the stator (4) as a function of angular position (φ) of the rotor (6) with respect to the stator (4).

## Patentansprüche

1. Mikropumpe (1), die einen Stator (4) und einen Rotor (6) umfasst, der axial und drehbar in Bezug auf den Stator beweglich ist, wobei der Stator (4) eine Kolbenkammer (10) umfasst, die eine radiale Oberfläche (30), einen Einlass (3), der eine Einlassmündung (28) umfasst, die in der radialen Oberfläche gebildet ist, und einen Auslass (5) umfasst, der eine Auslassmündung (29) umfasst, die in der radialen Oberfläche gebildet ist, wobei der Rotor (6) einen Kolbenabschnitt (13) umfasst, der eine radiale Oberfläche (16), eine Endseite (15) und einen nicht achsensymmetrischen Einschnitt (14) umfasst, der zwischen der radialen Oberfläche (16) und der Endseite (15) des Kolbenabschnitts (13) gebildet ist, wobei der Einschnitt (14) so eingerichtet ist, dass er die Einlassmündung (28) zumindest teilweise über einen Bereich von Winkelpositionen des Rotors in Bezug auf den Stator überlappt, die einer Pumpeneinlassphase entsprechen, und so eingerichtet ist, dass er die Auslassmündung (29) zumindest teilweise über einen Bereich von Winkelpositionen des Rotors in Bezug auf den Stator überlappt, die einer Pumpenauslaufphase entsprechen, und wobei die radiale Oberfläche (16) des Kolbenabschnitts (13) dazu ausgestaltet ist, die Auslassmündung (29) während der Pumpeneinlassphase, die Einlassmündung (28) während der Pumpenauslassphase und sowohl die Einlass- als auch die Auslassmündung (28) zwischen der Pumpeneinlass- und der Pumpenauslassphase zu schließen, wobei der Stator (4) eine flexible elastische Membran (7) umfasst, die den Kolbenabschnitt (13) dichtend von der Kolbenkammer (10) trennt, wobei der Kolbenabschnitt (13) geformt ist und die Membran dazu ausgestaltet ist, die Membran während eines vollständigen Pumpenzyklus elastisch auf eine formschlüssige, elastisch gespannte Weise über dem Einschnitt (14), der Endseite (15) und der radialen Oberfläche (16) des Kolbenabschnitts (13) anzupassen.

2. Mikropumpe nach Anspruch 1, wobei die flexible elastische Membran (7) eine einzelne Schicht oder eine Vielzahl von Schichten aus einem flexiblen Material umfasst.

3. Mikropumpe nach dem direkt vorhergehenden Anspruch, wobei das flexible Material Silikonkautschuk oder thermoplastisches Elastomer ist.

4. Mikropumpe nach einem der zwei direkt vorhergehenden Ansprüche, wobei die Dicke der einzelnen Schicht oder jeder der Vielzahl von Schichten zwischen 0,1 mm und 1 mm beträgt.

5. Mikropumpe nach einem der drei direkt vorhergehenden Ansprüche, wobei die flexible elastische Membran (7) die Vielzahl von Schichten umfasst und die Pumpe ein Schmiermittel zwischen mindestens zwei der Vielzahl von Schichten umfasst.

6. Mikropumpe nach einem der vorhergehenden Ansprüche, die ein Schmiermittel zwischen dem Kolbenabschnitt (13) und der flexiblen elastischen Membran (7) umfasst.

7. Mikropumpe nach einem der vorhergehenden Ansprüche, wobei das Verhältnis des Kolbenhubs zum Durchmesser des Kolbenabschnitts (13) kleiner als 0,5, vorzugsweise kleiner als 0,2, ist.

8. Mikropumpe nach einem der vorhergehenden Ansprüche, wobei der Stator (4) einen Kappenabschnitt (4b) und einen Basisabschnitt (4a) umfasst und wobei ein Randabschnitt der Membran (7) zwischen dem Kappenabschnitt (4b) und dem Basisabschnitt (4a) befestigt ist.

9. Mikropumpe nach dem direkt vorhergehenden Anspruch, wobei der Kappenabschnitt (4b) durch eine Schnappverschlussanordnung an dem Basisabschnitt (4a) befestigt ist.

10. Mikropumpe nach einem der vorhergehenden Ansprüche, ferner umfassend Dichtungsrippen (17, 18, 23, 24), die von dem Rotor (6) oder der Oberfläche der Kolbenkammer (10) hervorstehen und dazu ausgestaltet sind, die flexible elastische Membran (7) dichtend zusammenzudrücken und die Kolbenkammer zumindest über der Einlass- und der Auslassmündung abzudichten.

11. Mikropumpe nach dem direkt vorhergehenden Anspruch, wobei die Dichtungsrippen mindestens eine untere Dichtungsrippe (18), die von dem Rotor hervorsteht und dazu ausgestaltet ist, die elastische Membran (7) während einer offenen Phase des Einlasses (3) unter der Auslassmündung (29) und über der Einlassmündung (28) beziehungsweise während einer offenen Phase des Auslasses (5) unter der Einlassmündung (28) und über der Auslassmündung (29) dichtend zusammenzudrücken.

12. Mikropumpe nach Anspruch 10, wobei die Dichtungsrippen Dichtungsrippen (24) umfassen, die von der radialen Oberfläche (30) der Kolbenkammer (10) die Einlassmündung (28) und die Auslassmündung (29) umgebend hervorstehen.

13. Mikropumpe nach einem der vorhergehenden Ansprüche, umfassend ein Nockensystem (8), das eine axiale Position (z) des Rotors (6) in Bezug auf den Stator (4) als eine Funktion einer Winkelposition (φ) des Rotors (6) in Bezug auf den Stator (4) definiert.

## Revendications

1. Micropompe (1) comprenant un stator (4) et un rotor (6) mobile de manière axiale et rotative par rapport au stator, le stator (4) comprenant une chambre de piston (10) comprenant une surface radiale (30), une entrée (3) comprenant une bouche d'entrée (28) formée dans la surface radiale et une sortie (5) comprenant une bouche de sortie (29) formée dans la surface radiale, le rotor (6) comprenant une partie de piston (13) comprenant une surface radiale (16), une face d'extrémité (15) et une indentation non axisymétrique (14) formée entre la surface radiale (16) et la face d'extrémité (15) de la partie de piston (13), dans laquelle l'indentation (14) est agencée pour recouvrir au moins partiellement la bouche d'entrée (28) sur une plage de positions angulaires du rotor par rapport au stator correspondant à une phase d'admission de pompe, et agencée pour recouvrir au moins partiellement la bouche de sortie (29) sur une plage de positions angulaires du rotor par rapport au stator correspondant à une phase d'expulsion de pompe, et dans laquelle la surface radiale (16) de la partie de piston (13) est configurée pour fermer la bouche de sortie (29) pendant la phase d'admission de pompe, la bouche d'entrée (28) pendant la phase d'expulsion de pompe et à la fois les bouches d'entrée et de sortie (28) entre les phases d'admission et d'expulsion de pompe, dans laquelle le stator (4) comprend une membrane élastique flexible (7) séparant, de manière étanche, la partie de piston (13) de la chambre de piston (10), dans laquelle la partie de piston (13) est façonnée, et la membrane est configurée pour épouser élastiquement la membrane de façon élastiquement tendue et ajustée à la forme sur l'indentation (14), la face d'extrémité (15) et la surface radiale (16) de la partie de piston (13) pendant tout le cycle de la pompe.

2. Micropompe selon la revendication 1, dans laquelle la membrane élastique flexible (7) comprend une seule couche ou une pluralité de couches d'un matériau flexible.

3. Micropompe selon la revendication directement précédente, dans laquelle le matériau flexible est du caoutchouc de silicone ou un élastomère thermoplastique.

4. Micropompe selon l'une quelconque des deux revendications directement précédentes, dans laquelle l'épaisseur de la couche unique ou de chacune de la pluralité de couches est comprise entre 0,1 mm et 1 mm.

5. Micropompe selon l'une quelconque des trois revendications directement précédentes, dans laquelle la membrane élastique flexible (7) comprend ladite pluralité de couches et la pompe comprend un lubrifiant entre au moins deux de la pluralité de couches.

6. Micropompe selon l'une quelconque des revendications précédentes, comprenant un lubrifiant entre la partie de piston (13) et la membrane élastique flexible (7).

7. Micropompe selon l'une quelconque des revendications précédentes, dans laquelle le rapport de la course de piston sur le diamètre de la partie de piston (13) est inférieur à 0,5, de préférence inférieur à 0,2.

8. Micropompe selon l'une quelconque des revendications précédentes, dans laquelle le stator (4) comprend une partie de capuchon (4b) et une partie de base (4a) et dans laquelle une partie de rebord de la membrane (7) est fixée entre la partie de capuchon (4b) et la partie de base (4a).

9. Micropompe selon la revendication directement précédente, dans laquelle la partie de capuchon (4b) est fixée à la partie de base (4a) par un agencement de verrouillage par encliquetage.

10. Micropompe selon l'une quelconque des revendications précédentes, comprenant en outre des nervures d'étanchéité (17, 18, 23, 24) faisant saillie du rotor (6) ou de la surface de la chambre de piston (10), configurée pour pincer, de manière étanche, la membrane élastique flexible (7) et sceller la chambre de piston au moins au-dessus des bouches d'entrée et de sortie.

11. Micropompe selon la revendication directement précédente, dans laquelle les nervures d'étanchéité comprennent au moins une nervure d'étanchéité inférieure (18) faisant saillie du rotor configurée pour pincer, de manière étanche, la membrane élastique (7) au-dessous de la bouche de sortie (29) et au-dessus de la bouche d'entrée (28) pendant une phase ouverte de l'entrée (3), respectivement au-dessous de la bouche d'entrée (28) et au-dessus de la bouche de sortie (29) pendant une phase ouverte de la sortie (5).

12. Micropompe selon la revendication 10, dans laquelle les nervures d'étanchéité comprennent des nervures d'étanchéité (24) faisant saillie de la surface radiale (30) de la chambre de piston (10) entourant la bouche d'entrée (28) et la bouche de sortie (29).

13. Micropompe selon l'une quelconque des revendications précédentes, comprenant un système de came (8) qui définit une position axiale (z) du rotor (6) par rapport au stator (4) en fonction de la position angulaire (φ) du rotor (6) par rapport au stator (4).
